# EUROPEAN PATENT APPLICATION

(11) **EP 4 613 734 A1**
(43) Date of publication of application: **10.09.2025**
(21) Application number: 23884739.6
(22) Date of filing: 27.10.2023
(51) Int. Cl.: C07C 209/16, C07C 209/86, C07C 211/12

(54) **METHOD FOR PREPARING HEXAMETHYLENEDIAMINE**

(30) Priority: 31.10.2022 CN 202211351228; 31.10.2022 CN 202211352163; 20.10.2023 CN 202311369336
(71) Applicant: CHINA PETROLEUM & CHEMICAL CORPORATION, Chaoyang District Beijing 100728 (CN); Sinopec (Beijing) Research Institute of Chemical Industry Co., Ltd., Beijing 100013 (CN)
(72) Inventor: LUO, Shujuan, Beijing 100013 (CN); LI, Yan, Beijing 100013 (CN); PENG, Hui, Beijing 100013 (CN); WANG, Guoqing, Beijing 100013 (CN); LI, Dongfeng, Beijing 100013 (CN); SHU, Zhan, Beijing 100013 (CN); SUN, Ruliu, Beijing 100013 (CN); LI, Yulong, Beijing 100013 (CN); LIU, Zhixin, Beijing 100013 (CN); GUO, Liang, Beijing 100013 (CN); LI, Yi, Beijing 100013 (CN); SHI, Qian, Beijing 100013 (CN); TIAN, Jun, Beijing 100013 (CN); CHANG, Dashan, Beijing 100013 (CN); FAN, Xiaozhe, Beijing 100013 (CN); ZHAO, Meng, Beijing 100013 (CN); QU, Yuping, Beijing 100013 (CN); HU, Zhiyan, Beijing 100013 (CN); LI, Shouzhen, Beijing 100013 (CN); WANG, Chao, Beijing 100013 (CN); SHAO, Huawei, Beijing 100013 (CN)
(74) Representative: Hoffmann Eitle
(86) International application number: PCT/CN2023/126962
(87) International publication number: WO 2024/093807

(57) **Abstract**

This invention discloses a method for preparing hexamethylenediamine, comprising the following steps: (1) under ammoniation reaction conditions, optionally in the presence of a solvent, subjecting hexanediol and ammonia to an ammoniation reaction to obtain an ammoniation reaction product; (2) separating and obtaining from the ammoniation reaction product a stream containing hexamethyleneimine and water, dehydrating the stream containing hexamethyleneimine and water to obtain a dehydrated stream containing hexamethyleneimine, and returning at least part of the dehydrated stream containing hexamethyleneimine as a circulating stream to the step (1), wherein a water content in the dehydrated stream containing hexamethyleneimine is less than 3 wt%, preferably less than 1.5 wt%. The invention can not only improve the yield of hexamethylenediamine but can also prolong the service life of the catalyst.

## Description

### Technical field

The invention relates to a technical field of hexamethylenediamine production, in particular to a method for preparing hexamethylenediamine.

### Background

Hexamethylenediamine, also known as 1,6-diaminohexane, is an important chemical raw material, which is mainly used to synthesize nylon-66 salt, nylon 610, 1,6-hexamethylene diisocyanate and other products. These products are used to manufacture fibers, resins, engineering plastics, etc. They have a wide range of uses and can be widely used in the fields of textile leathers, architectural coatings, machinery manufacturing and so on.

The main methods for industrial production of hexamethylenediamine include an adiponitrile method, a caprolactam method, an adipic acid method, a hexanediol ammoniation method, and the like.

The adiponitrile method is the most commonly used production method in industrial production. The method is divided into two approaches: a high-pressure method and a low-pressure method. The high-pressure method uses iron based and cobalt-copper based catalysts as the main catalysts, and the conversion rate can reach 95% or more, but the pressure is high and there is an extremely high requirement on equipments. The low-pressure method uses Raney nickel as the main catalyst, but there are problems related to the safety and stability of the Raney nickel catalyst, the supply of the adiponitrile raw material, etc.

In the caprolactam method, caprolactam is ammoniated with ammonia under the catalysis of a phosphate salt to produce 6-aminocapronitrile, which is hydrogenated to produce hexamethylenediamine. The key step of the method is the ammoniation of caprolactam. This method has been gradually abandoned because it is only applicable to a small-scale production and its production cost is high.

The adipic acid method is that adipic acid is ammoniated with ammonia, followed by dehydration to produce adiponitrile, which is hydrogenated to obtain hexamethylenediamine. This method has high production cost and a long process, thus is limited in process technology development.

The hexanediol method is the preparation of hexamethylenediamine by subjecting hexanediol and ammonia, as raw materials, to ammoniation reaction. Hexanediol is a colorless, transparent liquid with low toxicity and little harm to the environment and human body. The preparation of hexamethylenediamine using hexanediol as raw material is a green and environmentally friendly route. However, in the prior art, the yield of production of hexamethylenediamine by ammoniation of hexanediol is generally low. As calculated according to the disclosed data, the yield of hexamethylenediamine is usually less than 50%, and the economic efficiency is left to be improved.

CN114433122A discloses a catalyst for preparing 1,6-hexamethylenediamine by ammoniation of 1,6-hexanediol in the presence of hydrogen. In a fixed bed reactor, the molar ratio of hydrogen: ammonia: 1,6-hexanediol is 3:8:1, the volume space velocity of 1,6-hexanediol is 0.6h⁻¹, the reaction temperature is 205°C, the reaction pressure is 13MPa, the conversion rate of hexanediol is about between 80% and 92%, and the selectivity of hexamethylenediamine is about 42%-52%. According to the disclosed data, it is calculated that the yield of hexamethylenediamine is about 34%-48%.

JP2022112396A discloses a method for producing hexamethylenediamine from 1,6-hexanediol, comprising producing hexamethylenediamine by amination reaction of 1,6-hexanediol under mild conditions in the presence of a solid catalyst, wherein the catalyst comprises an active component containing a metal element selected from elements of Group 8, Group 9, Group 10 and Group 11 in the periodic table and a carrier containing one or more metal elements selected from rare earth elements, and elements of Group 4 and Group 5. The examples show that the yield of hexamethylenediamine is less than 40%.

In the method of producing hexamethylenediamine using hexanediol as raw material in the prior art, the conversion rate of hexanediol and the yield of hexamethylenediamine product are both low. Therefore, there is still a need for a method for preparing hexamethylenediamine, in which hexamethylenediamine can be produced from hexanediol in a high yield, thereby improving the process economy.

### Summary

The object of the invention is to provide a method for preparing hexamethylenediamine with high yield so as to overcome the above problems existing in the prior art.

In order to achieve the above object, the invention provides a method for preparing hexamethylenediamine, which comprises the following steps:
(1) under ammoniation reaction conditions, optionally in the presence of a solvent, subjecting hexanediol and ammonia to an ammoniation reaction to obtain an ammoniation reaction product;
(2) separating and obtaining from the ammoniation reaction product a stream containing hexamethyleneimine and water, dehydrating the stream containing hexamethyleneimine and water to obtain a dehydrated stream containing hexamethyleneimine, and returning at least part of the dehydrated stream containing hexamethyleneimine as a circulating stream to the step (1), wherein a water content of the dehydrated stream containing hexamethyleneimine is less than 3 wt%, preferably less than 1.5 wt%.

The method of the invention has the following beneficial effects:
(1) The method of the invention can improve not only the yield of hexamethylenediamine but also the service life of the catalyst by dehydrating the stream containing hexamethyleneimine obtained by separating the ammoniation reaction product, and then returning at least part of the dehydrated stream containing hexamethyleneimine as a circulating stream to the ammoniation reaction.
(2) The invention can improve not only the yield of hexamethylenediamine but also the service life of the catalyst by controlling the water content of the stream containing hexamethyleneimine returned to the ammoniation reaction as a circulating stream, for example, the water content of less than 3wt%, preferably less than 1.5wt%.
(3) The method of the invention can also solve the problem of system blockage, ensure a long period stable operation of the entire process, reduce the number of shutdowns and maintenances, and lower the operating temperature of the entire separation process. At the same time, the separation process of the invention does not need full-process insulation measures, thereby reducing production costs.
(4) The method for preparing hexamethylenediamine of the invention has the characteristics of simple process, high yield of hexamethylenediamine product, strong operability, easy control, long period operation, etc.

### Detailed description

The endpoint values of the ranges and any values disclosed herein are not limited to the precise ranges or values, and these ranges or values should be understood to include values approximating these ranges or values. For numerical ranges, one or more new numerical ranges may be obtained by combining the endpoint values of each range with each other, by combing the endpoint values of each range with individual point values, and by combining individual point values with each other, and these numerical ranges should be deemed to be specifically disclosed herein.

The invention provides a method for preparing hexamethylenediamine, which comprises the following steps:
(1) under ammoniation reaction conditions, optionally in the presence of a solvent, subjecting hexanediol and ammonia to an ammoniation reaction to obtain an ammoniation reaction product;
(2) separating and obtaining from the ammoniation reaction product a stream containing hexamethyleneimine and water, dehydrating the stream containing hexamethyleneimine and water to obtain a dehydrated stream containing hexamethyleneimine, and returning at least part of the dehydrated stream containing hexamethyleneimine as a circulating stream to the step (1); wherein a water content of the dehydrated stream containing hexamethyleneimine is less than 3 wt%, preferably less than 1.5 wt%.

It is unexpectedly discovered by the invention that, when the amount of water in the ammoniation reaction system increases, a hydrothermal reforming reaction may occur, affecting the crystal form of the catalyst carrier and the life of the catalyst, thereby affecting the conversion rate and yield of the ammoniation reaction. The invention finds that, the yield of hexamethylenediamine may be greatly improved by strictly controlling the water content in the circulating stream in the ammoniation reaction system, thereby controlling the content of water entering the ammoniation reactor. According to the invention, the water content in the dehydrated stream containing hexamethyleneimine may be less than 3wt%, preferably less than 1.5wt%, and more preferably less than 1wt%.

According to the invention, a solvent is not essential, but optionally used in the ammoniation reaction of hexanediol and ammonia, thus the ammoniation reaction may be carried out in a solvent or in the absence of a solvent. **In** the prior art, ammoniation reaction is usually carried out at normal pressure or low pressure, and the ammonia is in a gas phase state, so a solvent needs to be added to dissolve the ammonia gas in order to effectively carry out the ammoniation reaction. In the invention, the pressure of the ammoniation reaction is relatively high, and the ammonia is liquefied to be in a liquid phase state, thus the ammoniation reaction of hexanediol and liquid ammonia may be carried out in the absence of a solvent. The solvent added in the invention is a solvent in which the solubility of the ammoniation reaction raw materials and reaction products such as hexanediol, hexamethylenediamine, aminohexanol and hexamethyleneimine is greater than 0.1g/g, such as at least one of tetrahydrofuran, 1,4-dioxane, n-hexane, cyclohexane, tert-butanol, tetramethylene sulfone, glycerol, etc.

According to the invention, in the ammoniation reaction of hexanediol and ammonia, the molar ratio of ammonia to hexanediol is 10-60:1, preferably 15-55:1, such as 18-50:1, 22-43:1, and 22-38:1. The ammoniation reaction is carried out in the presence of hydrogen, and the molar ratio of hydrogen to hexanediol is 0.05-8:1, preferably 0.08-7:1, preferably 0.09-6:1, preferably 0.1-5:1, preferably 0.2-4:1, preferably 0.3-4:1, more preferably 0.4-3:1, for example 0.4-5:1, and 0.4-4:1. The molar ratio of ammonia, hydrogen and hexanediol refers to the molar ratio in the mixture at the inlet of the ammoniation reactor.

According to the invention, in the ammoniation reaction of hexanediol and ammonia, the temperature of the ammoniation reaction is 100-220°C, preferably 110-210°C, preferably 120-210°C, preferably 130-200°C, for example 110-200°C, and 120-190°C. The pressure of the ammoniation reaction is 5-20 MPaG, preferably 6-19 MPaG, preferably 7-17 MPaG, more preferably 8-15 MPaG, such as 7-18 MPaG, and 8-14 MPaG. The liquid volume space velocity of the fresh hexanediol is 0.05-10h⁻¹, preferably 0.05-8h⁻¹, preferably 0.05-7h⁻¹, preferably 0.07-5h⁻¹, preferably 0.08-4h⁻¹, and more preferably 0.09-3.9h⁻¹.

The invention does not specifically limit the catalyst used in the ammoniation reaction, and all catalysts suitable for the ammoniation of hexanediol may be used, for example, the catalysts disclosed in patent applications such as CN114433087A and CN114433121A may be used. Preferably, the catalyst for the ammoniation reaction comprises a carrier and an active component supported on the carrier as well as optionally an auxiliary agent, wherein the carrier comprises a doping element, alumina and optional other carriers, the other carriers are selected from silicon oxide and/or molecular sieves, and the doping element is selected from at least one of boron, fluorine, phosphorus, sulfur and selenium. The pore volume of the carrier with a pore diameter in the range of 7-27 nm accounts for more than 65% of the pore volume of the carrier. The active component is cobalt and/or nickel.

According to the ammoniation reaction catalyst of the invention, preferably, the carrier is alumina incorporated with silicon oxide and/or molecular sieve. A content of the alumina carrier in the carrier is not less than 70wt%, preferably 75-100wt%.

According to the ammoniation reaction catalyst of the invention, preferably, a content of the doping element in the carrier accounts for 0.05-6wt%, more preferably 0.08-4wt% of the total weight of the carrier in terms of components other than the doping element. The components other than the doping element mainly refer to the alumina and the optional other carriers in the carrier.

According to the ammoniation reaction catalyst of the invention, preferably, the doping element in the carrier is doped in the form of at least one of borate ions, fluoride ions, phosphate ions, sulfate ions and selenate ions. Since the doped doping elements are introduced during the preparation process of the carrier, the doped elements are mainly present in the bulk phase of the carrier.

According to the ammoniation reaction catalyst of the invention, preferably, the pore volume of the carrier with a pore diameter in the range of 7-27 nm accounts for more than 65%, preferably 70-90% of the pore volume of the carrier, the pore volume of the carrier with a pore diameter less than 7 nm accounts for 0-10% of the pore volume of the carrier, and the pore volume of the carrier with a pore diameter greater than 27 nm accounts for 18-32% of the pore volume of the carrier.

According to the ammoniation reaction catalyst of the invention, preferably, the L acid of the carrier accounts for greater than or equal to 85%, more preferably 85-98% of the sum of the L acid and B acid.

According to the ammoniation reaction catalyst of the invention, preferably, the specific surface area of the carrier is 120-210 m²/g, and the pore volume of the carrier is 0.43-1.1 ml/g.

According to the ammoniation reaction catalyst of the invention, preferably, a content of the active component may be 8-44 g, preferably 12-37 g per 100 g of the carrier in terms of components other than the doping element.

According to the ammoniation reaction catalyst of the invention, preferably, a content of the auxiliary agent may be 0-10 g, preferably 0.5-6 g per 100 g of the carrier in terms of components other than the doping element.

According to the ammoniation reaction catalyst of the invention, preferably, the auxiliary agent may be selected from at least one of elements of Group VIB, Group VIIB, Group IB, Group IIB and lanthanides, preferably at least one of Cr, Mo, W, Mn, Re, Cu, Ag, Au, Zn, La and Ce.

There are no restrictions on the type of ammoniation reactor used for the ammoniation reaction, and any reactor that may ensure stable operation of the reaction may be used, such as a fixed bed reactor, a high-pressure reactor, and a fluidized bed reactor.

According to the invention, from an overall consideration of the system energy consumption, the yield of hexamethylenediamine and the service life of the catalyst, preferably, the weight ratio of the circulating stream returned to the step (1) to the fresh hexanediol is 0.1-30:1, preferably 0.3-26:1, more preferably 0.5-20:1, for example 0.1-24:1, 0.4-21:1, 0.1-13:1, and 0.3-10:1.

It should be noted that, at the beginning of the reaction, the amount of hexamethyleneimine generated by the ammoniation reaction is small and cannot meet the above-defined weight ratio of the circulating stream to the fresh hexanediol. Thus in the initial stage of the reaction, all the hexamethyleneimine is returned to the ammoniation reaction. During the stable operation stage of the reaction, the amount of hexamethyleneimine accumulatively generated by the ammoniation reaction is sufficiently high, and at this time, it is only necessary to ensure that the returned circulating stream meets the above-defined weight ratio. The same is true for the aminohexanol generated by the ammoniation reaction. The weight ratio of the circulating stream returned to the step (1) to the fresh hexanediol given in the examples of the description is the mass ratio in the stable operation stage of the reaction.

According to the invention, the dehydration for the stream containing hexamethyleneimine and water may be a dehydration commonly used in the art, but in order to further reduce the water content in the stream containing hexamethyleneimine, preferably, the dehydration includes at least one of membrane separation dehydration, azeotropic distillation dehydration, extractive distillation dehydration, adsorption dehydration, reverse osmosis dehydration, biological treatment dehydration, pressure swing distillation dehydration, etc.

According to a preferred embodiment of the invention, the dehydration for the stream containing hexamethyleneimine and water may be the membrane separation dehydration, and the operating conditions of the membrane separation dehydration include: a membrane inlet pressure of 0-5MPaG, such as 1-4MPaG, and a temperature of 10-350°C, such as 10-200°C, such as 15-190°C.

According to a preferred embodiment of the invention, the dehydration for the stream containing hexamethyleneimine and water may be the azeotropic distillation dehydration. The azeotropic distillation dehydration is carried out in a distillation column, and the operating conditions of the distillation column include: a weight ratio of an azeotropic agent to the stream containing hexamethyleneimine of 10-100:1, a bottom temperature of 120-200°C, a top temperature of 80-150°C, a reflux ratio of 0.1-20, a top operating pressure of 0-3MPaG, and a plate number of 10-80; and the azeotropic agent may be at least one of cyclohexane, n-hexane, trimethylpentane, 1-methyl-4-isopropylbenzene, dioxane, phenol, cresol, dibutyl ether, diamyl ether and diisoamyl ether.

According to the invention, the step (2) further comprises separating and obtaining a stream containing aminohexanol from the ammoniation reaction product, and returning the stream containing aminohexanol together with the dehydrated stream containing hexamethyleneimine as a circulating stream to the step (1) to further improve the yield of hexamethylenediamine. The stream containing aminohexanol may also contain, for example, 0-30 wt% of hexanediol, 0-2 wt% of hexamethylenediamine, and a small amount of other components (e.g., heavy components).

According to the invention, preferably, the weight ratio of aminohexanol to hexamethyleneimine in the circulating stream is 0.1-8:1, for example 0.1-7:1, for example 0.12-7:1 (for example, 0.12:1, 0.5:1, 0.6:1, 0.7:1, 0.8:1, 0.9:1, 1:1, 2:1, 3:1, 4:1, 5:1, 6:1, 7:1, and a range constituted by any two points above).

It should be noted that, when the stream containing aminohexanol and the dehydrated stream containing hexamethyleneimine are returned to the step (1) together as the circulating stream, since the amount of hexamethyleneimine and aminohexanol generated by the ammoniation reaction is small at the beginning of the reaction and cannot meet the above-defined weight ratio of the circulating stream to hexanediol, the dehydrated stream containing hexamethyleneimine and the stream containing aminohexanol are all returned to the ammoniation reaction in the initial stage of the reaction. During the stable operation stage of the reaction, the accumulative amount of hexamethyleneimine and aminohexanol generated by the ammoniation reaction is sufficiently high, and at this time, it is only necessary to ensure that the returned circulating stream meets the above-defined weight ratio. The weight ratio of the circulating stream returned to the ammoniation reaction to the fresh hexanediol given in the examples of the description is the weight ratio in the stable operation stage of the reaction.

According to the invention, preferably, the conditions for separating the ammoniation reaction product are such that a content of hexamethyleneimine in the stream containing hexamethyleneimine and water is 35-95wt%, more preferably 40-85wt%, most preferably 50-80wt%; and the water content is 5-65wt%, more preferably 20-50wt%, most preferably 30-40wt%.

Preferably, the approach for separating the ammoniation reaction product is as follows: subjecting the ammoniation reaction product to a first separation to obtain a stream containing hexamethyleneimine and water, followed by a second separation to obtain a hexamethylenediamine product and a stream containing aminohexanol; more preferably, the first separation and the second separation each independently include at least one of distillation, membrane separation and pressure swing adsorption.

Preferably, the approach for separating the ammoniation reaction product is as follows: subjecting the ammoniation reaction product to a first separation to obtain a stream containing hexamethyleneimine and water, followed by a second separation to obtain a hexamethylenediamine product, and followed by a third separation to obtain a stream containing aminohexanol and heavy components; more preferably, the first separation, the second separation and the third separation each independently include at least one of distillation, membrane separation and pressure swing adsorption.

In the invention, for example, when distillation is used, the conditions for the first separation may include: a theoretical plate number of 10-80 (e.g., 10, 20, 30, 40, 50, 60, 70, 80, and a range constituted by any point values above), an operating pressure of 1-600 KPa (absolute pressure, e.g., 1 KPa, 10 KPa, 20 KPa, 30 KPa, 40 KPa, 50 KPa, 100 KPa, 200 KPa, 300 KPa, 400 KPa, 500 KPa, 600 KPa, and a range constituted by any point values above), a column bottom temperature of 100-300°C, and a reflux ratio of 0.1-15; the conditions for the second separation may include: a theoretical plate number of 10-80 (e.g., 10, 20, 30, 40, 50, 60, 70, 80, and a range constituted by any point values above), an operating pressure of 1-600 KPa (absolute pressure, for example, 1KPa, 10 KPa, 20 KPa, 30 KPa, 40 KPa, 50 KPa, 100 KPa, 200 Kpa, 300 KPa, 400 KPa, 500 KPa, 600 KPa, and a range constituted by any point values above), a column bottom temperature of 130-380°C, and a reflux ratio of 0.1-20; and the conditions for the third separation may include: a theoretical plate number of 10-100 (e.g., 10, 20, 30, 40, 50, 60, 70, 80, 90, 100, and a range constituted by any point values above), an operating pressure of 1-1100 KPa (absolute pressure, e.g., 1 KPa, 10 KPa, 20 KPa, 30 KPa, 40 KPa, 50 KPa, 100 KPa, 200 KPa, 300 KPa, 400 KPa, 500 KPa, 600 KPa, 700 KPa, 800 KPa, 900 KPa, 1000 KPa, 1100 KPa, and a range constituted by any point values above), a column bottom temperature of 150-400°C, and a reflux ratio of 0.1-20. The separation conditions of the invention may make the purity of the hexamethylenediamine product above 99.7wt%.

According to the invention, preferably, the step (2) further comprises pre-separating the ammoniation reaction product before separating the stream containing hexamethyleneimine and the hexamethylenediamine product in the ammoniation reaction product, to recover hydrogen and ammonia in the ammoniation reaction product; wherein the recovered hydrogen and ammonia are first condensed to obtain circulating ammonia (liquid phase), and then compressed to obtain circulating hydrogen (gas phase), and the circulating hydrogen and ammonia are returned to the ammoniation reaction; more preferably, the pre-separation method comprises at least one of flash evaporation, distillation and stripping. The pre-separation method may also employ other methods capable of recovering circulating hydrogen and ammonia from the ammoniation reaction product.

To ensure the effective utilization of hydrogen and ammonia and reduce the overall energy consumption of the process, preferably, the pre-separation conditions are such that the recovery rate of hydrogen is greater than 99% and the recovery rate of ammonia is greater than 98%. In the invention, the pre-separation method may adopt a multi-stage vacuum flash evaporation, the multi-stage flash evaporation may include at least two stages of flash evaporation, the pressure of the flash evaporation decreases gradually with a gradient of 1-5 MPa, and the pressure of the last stage of the flash evaporation is 0.5-1 MPaG. For example, a four-stage flash evaporation is adopted, and the flash evaporation pressures of the first stage to the fourth stage are 7-10 MPaG, 4-7 MPaG, 1-4 MPaG, and 0.1-1 MPaG, respectively. The pre-separation method may also adopt a combination of flash evaporation and distillation, for example, a combination of at least one stage of flash evaporation, such as a three-stage flash evaporation, and distillation, that is, the ammoniation reaction product is first subjected to a three-stage flash evaporation, the flash evaporation pressures of the first stage to the third stage are 7-10 MPaG, 4-7 MPaG, and 1-3 MPaG, respectively, and the liquid phase after the last stage of vacuum flash evaporation enters a distillation column for distillation. The conditions for operating the distillation column include a theoretical plate number of 5-30 and a top operating pressure of -0.05 MPaG to 10 MPaG, for example, 0.1 MPaG to 10 MPaG. Since a part of ammonia and hydrogen will be consumed during the ammoniation reaction, and a small amount of hydrogen and ammonia will be lost in the recovery process of hydrogen and ammonia, it is necessary to supplement fresh ammonia and hydrogen during the ammoniation reaction to maintain the molar ratio of hexanediol (including freshly supplemented hexanediol and hexanediol in the circulating stream), ammonia and hydrogen in the mixture at the feed port of the ammoniation reactor.

In the invention, hexamethylenediamine is 1,6-hexamethylenediamine, hexanediol is 1,6- hexanediol, hexamethyleneimine is homopiperidine, aminohexanol is 6-amino-1-hexanol, and the main components of the heavy components are bis(hexamethylene)triamine and amines substance with carbon number of 12 or more.

### The presence of the solvent in the ammoniation reaction

In an embodiment of the invention, in the method for preparing hexamethylenediamine,
step (1) comprises: under ammoniation reaction conditions, subjecting hexanediol and ammonia to the ammoniation reaction in the solvent to obtain an ammoniation reaction product;
step (2) comprises: separating and obtaining from the ammoniation reaction product a stream containing hexamethyleneimine, water and the solvent, dehydrating the stream containing hexamethyleneimine, water and the solvent to obtain a dehydrated stream containing hexamethyleneimine and the solvent, and returning at least part of the dehydrated stream containing hexamethyleneimine and the solvent as a circulating stream to the step (1).

Introducing the solvent into the ammoniation reaction may maintain a uniform temperature distribution of the system, which is conducive to reducing reaction hot spots, helps to improve the stability of the reaction, and ensures that no solid phase crystallization and separation occur throughout the system, reducing the operating temperature of each equipment in the device, and avoiding the huge investment brought by the device insulation. In addition, introducing the solvent may effectively dissolve hexanediol, and by-products such as hexamethyleneimine and aminohexanol produced by the ammoniation reaction, which helps to solve the problem of pipeline blockage during the entire operation cycle, especially in the separation process.

In the invention, the solvent is introduced at a one-time time (with hexanediol in the initial stage of the reaction). In the initial stage of the reaction (before returning the circulating stream to the ammoniation reaction), the molar ratio of the solvent to hexanediol is 0.1-10:1, preferably 0.15-10:1, more preferably 0.25-9:1. However, it is understandable that the solvent will be lost during the circulation and purification process. In order to ensure the amount of circulating solvent in the system, a small amount of fresh solvent may be supplemented during the solvent circulation process to maintain a constant amount of the solvent in the system. According to the invention, the solvent is a solvent in which the solubility of the ammoniation reaction raw materials and reaction products such as hexanediol, hexamethylenediamine, aminohexanol and hexamethyleneimine is greater than 0.1g/g, such as at least one of tetrahydrofuran, 1,4-dioxane, n-hexane, cyclohexane, tert-butanol, tetramethylene sulfone, glycerol, etc.

Preferably, the water content in the dehydrated stream containing hexamethyleneimine and the solvent is less than 3wt%, preferably less than 1.5wt%, more preferably less than 1wt%.

Preferably, in the step (1), the molar ratio of ammonia to hexanediol is 18-55:1, preferably 22-43:1; the ammoniation reaction is carried out in the presence of hydrogen, and the molar ratio of hydrogen to hexanediol is 0.08-7:1, preferably 0.4-4:1.

Preferably, the temperature of the ammoniation reaction is 110-200°C, preferably 120-190°C; the pressure of the ammoniation reaction is 7-18 MPaG, preferably 8-14 MPaG, and the liquid volume space velocity of the fresh hexanediol is 0.05-8 h⁻¹, preferably 0.08-4 h⁻¹.

Preferably, the weight ratio of the solvent to hexamethyleneimine in the circulating stream is 1-11:1 (e.g., 1:1, 2:1, 3:1, 4:1, 5:1, 6:1, 7:1, 8:1, 9:1, 10:1, 11:1, and a range constituted by any two points above). The content of hexamethyleneimine in the circulating stream is 5-75wt%, such as 5-50wt%. Preferably, the weight ratio of the circulating stream to the fresh hexanediol is 0.1-24:1, preferably 0.4-21:1.

Preferably, the dehydration for the stream containing hexamethyleneimine, water and the solvent includes at least one of membrane separation dehydration, azeotropic distillation dehydration, extractive distillation dehydration, adsorption dehydration, reverse osmosis dehydration, biological treatment dehydration and pressure swing distillation dehydration.

According to a preferred embodiment of the invention, the process of dehydrating the stream containing hexamethyleneimine, water and the solvent comprises: subjecting the stream containing hexamethyleneimine, water and the solvent to membrane separation dehydration at a membrane inlet pressure of 1-5 MPa and 15-200°C.

According to a preferred embodiment of the invention, the process of dehydrating the stream containing hexamethyleneimine, water and the solvent comprises: feeding the stream containing hexamethyleneimine, water and the solvent to a distillation column, obtaining a stream containing the solvent and water at the top of the column, and obtaining a stream containing hexamethyleneimine at the bottom of the column; feeding the stream containing the solvent and water to a high-pressure distillation column, obtaining an azeotrope of the solvent and water at the top of the column, and obtaining a solvent stream at the bottom of the column; and then feeding the azeotrope of the solvent and water to a low-pressure distillation column, and returning the stream obtained at the top of the column to the high-pressure distillation column. The conditions for operating the distillation column may include a bottom temperature of 80-150°C, a top temperature of 30-90°C, a reflux ratio of 1-20, a top operating pressure of -0.1 MPaG to 2 MPaG, and a plate number of 10-40. The conditions for operating the high-pressure distillation column may include a bottom temperature of 180-280°C, a top temperature of 130-230°C, a reflux ratio of 0.1-5, a top operating pressure of 0-4 MPaG, and a plate number of 10-40. The conditions for operating the low-pressure distillation column may include a bottom temperature of 70-150°C, a top temperature of 40-120°C, a reflux ratio of 0.5-10, a top operating pressure of -0.1 MPaG to 3 MPaG, and a plate number of 10-30. By using a high-pressure distillation column and a low-pressure distillation column in combination, the invention may effectively remove water from the stream containing hexamethyleneimine, water and the solvent.

Preferably, the step (2) further comprises separating and obtaining a stream containing aminohexanol from the ammoniation reaction product, and returning the stream containing aminohexanol together with the dehydrated stream containing hexamethyleneimine and the solvent as a circulating stream to the step (1). The stream containing aminohexanol may also contain hexanediol, hexamethylenediamine, and a small amount of other components (e.g., heavy components).

Preferably, the weight ratio of aminohexanol to hexamethyleneimine in the circulating stream is 0.12-7:1 (e.g., 0.12:1, 0.5:1, 0.6:1, 0.7:1, 0.8:1, 0.9:1, 1:1, 3:1, 5:1, 7:1, and a range constitute by any two points above).

According to a preferred embodiment of the invention, the method for separating the ammoniation reaction product comprises: subjecting the ammoniation reaction product to a first separation to obtain the stream containing hexamethyleneimine, water and the solvent, followed by a second separation to obtain a hexamethylenediamine product and a stream containing aminohexanol. Preferably, the first separation is carried out in a first distillation column, and the conditions for operating the first distillation column include: a bottom temperature of 130-300°C, a top temperature of 40-70°C, a reflux ratio of 0.1-10, a top operating pressure of -0.1 MPaG to 1 MPaG, and a theoretical plate number of 5-40; the second separation is carried out in a second distillation column, and the conditions for operating the second distillation column include: a bottom temperature of 130-380°C, a top temperature of 80-190°C, a reflux ratio of 0.1-20, a top operating pressure of -0.1 MPaG to 1 MPaG, and a theoretical plate number of 10-100.

According to a preferred embodiment of the invention, the method for separating the ammoniation reaction product comprises: subjecting the ammoniation reaction product to a first separation to obtain the stream containing hexamethyleneimine, water and the solvent, followed by a second separation to obtain a hexamethylenediamine product, and followed by a third separation to obtain a stream containing aminohexanol and heavy components. Preferably, the first separation is carried out in a first distillation column, and the conditions for operating the first distillation column include: a bottom temperature of 130-300°C, a top temperature of 30-80°C, a reflux ratio of 0.1-10, a top operating pressure of -0.1 MPaG to 1 MPaG, and a plate number of 8-45; the second separation is carried out in a second distillation column, and the conditions for operating the second distillation column include: a bottom temperature of 130-380°C, a top temperature of 85-185°C, a reflux ratio of 0.1-20, a top operating pressure of -0.1 MPaG to 1 MPaG, and a plate number of 10-100; the third separation is carried out in a third distillation column, and the conditions for operating the third distillation column include: a bottom temperature of 150-380°C, a top temperature of 130-230°C, a reflux ratio of 0.1-15, a top operating pressure of -0.1 MPaG to 1MPaG, and a plate number of 10-100.

According to a preferred embodiment of the invention, the method for separating the ammoniation reaction product comprises: subjecting the ammoniation reaction product to a first separation to obtain a stream containing hexamethyleneimine and a remaining component of the first separation, then subjecting the remaining component of the first separation to a second separation to obtain a hexamethylenediamine product, a stream containing aminohexanol and a remaining component of the second separation, and then subjecting the remaining component of the second separation to a third separation to obtain a solvent stream and heavy components. Preferably, the first separation is carried out in a first distillation column, and the conditions for operating the first distillation column include: a bottom temperature of 200-280°C, a top temperature of 30-80°C, a reflux ratio of 0.5-10, a top operating pressure of -0.1 MPaG to 0.2 MPaG, and a plate number of 10-45; the second separation is carried out in a second distillation column, and the conditions for operating the second distillation column include: a bottom temperature of 200-310°C, a top temperature of 70-180°C, a reflux ratio of 0.5-15, a top operating pressure of -0.1 MPaG to 0.4 MPaG, and a plate number of 20-80; the third separation is carried out in a third distillation column, and the conditions for operating the third distillation column include: a bottom temperature of 200-380°C, a top temperature of 130-300°C, a reflux ratio of 0.5-20, a top operating pressure of -0.1 MPaG to 0.2 MPaG, and a plate number of 10-80.

Preferably, the method further comprises recovering hydrogen and ammonia in the ammoniation reaction product before separating the stream containing hexamethyleneimine, water and the solvent in the ammoniation reaction product. In the invention, the method for recovering hydrogen and ammonia in the ammoniation reaction product may adopt a multi-stage vacuum flash evaporation, the multi-stage flash evaporation may include at least two stages of flash evaporation, the pressure of the flash evaporation decreases gradually with a gradient of 1-5 MPa, and the pressure of the last stage of the flash evaporation is 0.1-2 MPaG. For example, a four-stage flash evaporation is adopted, the flash evaporation pressures of the first stage to the fourth stage are 7-10 MPaG, 4-7 MPaG, 2-4 MPaG, and 0.1-2 MPaG respectively. The method for recovering hydrogen and ammonia in the ammoniation reaction product may also adopt a combination of flash evaporation and distillation, where the ammoniation reaction product is subjected to at least one stage of flash evaporation, and the liquid phase after the last stage of the flash evaporation is further distilled. When a combination of one-stage flash evaporation and distillation is used, the pressure of the flash evaporation is 1-3 MPaG; when a combination of multi-stage flash evaporation and distillation is used, the pressure of the flash evaporation decreases gradually to 0.1-2 MPaG with a gradient of 0.1-10 MPa. For example, when a combination of three-stage flash evaporation and distillation is adopted, the ammoniation reaction product is first subjected to the three-stage flash evaporation, the flash evaporation pressures of the first stage to the third stage are 7-10 MPaG, 4-7 MPaG, and 1-3 MPaG, respectively, and the liquid phase after the last stage of the vacuum flash evaporation enters a distillation column, which is provided with 5-30 theoretical plates and has a top pressure of -0.05 MPaG to 3 MPaG. The recovery rate of ammonia at the top of the distillation column is greater than 98%. More preferably, each stage of the flash evaporation is equipped with a cooler and a gas-liquid separator, and the flash tank at each stage is connected in sequence with its equipped cooler and gas-liquid separator to form a circular connection. For example, the gas phase outlet of the first-stage flash tank is connected to the inlet of the condenser, the outlet of the condenser is connected to the inlet of the gas-liquid separator, and the liquid phase outlet of the gas-liquid separator is connected to the inlet of the first-stage flash tank. The gas phase at the top of the flash tank at each stage enters its respectively connected condenser to be cooled to 30-60°C, and the cooled stream is separated by the equipped gas-liquid separator, with the resultant gas phase being pressurized and then recycled back to the ammoniation reactor, and the resultant liquid phase being returned to the flash tank at the respective stage. In brief, the gas phase obtained from each stage of the flash evaporation is respectively cooled to 30-60°C, and the cooled stream is respectively subjected to gas-liquid separation, wherein the resultant liquid phase is returned to the flash tank at the respective stage, and the resultant gas phase is pressurized and then recycled back to the ammoniation reactor.

In the invention, it is understandable that since a part of ammonia and hydrogen are consumed in the ammoniation reaction, or a part of ammonia and hydrogen are released as a purge gas during recovery, it is also necessary to supplement fresh ammonia and hydrogen during the amination reaction to maintain the molar ratio of hexanediol (including freshly supplemented hexanediol, and hexanediol in the circulating stream), ammonia and hydrogen in the mixture at the feed port of the ammoniation reactor.

### Absence of the solvent in the ammoniation reaction

In an embodiment of the invention, in the method for preparing hexamethylenediamine,
step (1) comprises: under ammoniation reaction conditions, and in the absence of the solvent, subjecting hexanediol and ammonia to an ammoniation reaction to obtain an ammoniation reaction product;
step (2) comprises: separating and obtaining from the ammoniation reaction product a stream containing hexamethyleneimine and water, dehydrating the stream containing hexamethyleneimine and water to obtain a dehydrated stream containing hexamethyleneimine, and returning at least part of the dehydrated stream containing hexamethyleneimine as a circulating stream to the step (1); wherein a water content of the dehydrated stream containing hexamethyleneimine is less than 3 wt%, preferably less than 1.5 wt%.

Preferably, in the step (1), the molar ratio of ammonia to hexanediol is 18-50:1, more preferably 22-38:1, and the molar ratio of hydrogen to hexanediol is 0.08-6:1, more preferably 0.4-3:1. The molar ratio of ammonia, hydrogen and hexanediol refers to the molar ratio in the mixture at the inlet of the ammoniation reactor.

Preferably, the temperature of the ammoniation reaction is 120-210°C, preferably 130-200°C; the pressure of the ammoniation reaction is 7-17 MPaG, more preferably 8-15 MPaG, and the liquid volume space velocity of the fresh hexanediol is 0.05-7h⁻¹, more preferably 0.09-3.9h⁻¹.

Preferably, the weight ratio of the circulating stream to fresh hexanediol is 0.1-13:1, preferably 0.3-10:1.

Preferably, the step (2) further comprises separating and obtaining a stream containing aminohexanol from the ammoniation reaction product, and returning the stream containing aminohexanol together with the dehydrated stream containing hexamethyleneimine as a circulating stream to the step (1), wherein the weight ratio of aminohexanol to hexamethyleneimine in the circulating stream is 0.1-5:1, for example, 0.12:1, 0.5:1, 0.6:1, 0.7:1, 0.8:1, 0.9:1, 1:1, 3:1, 5:1.

### Examples

The invention will be described in detail below through examples. In the following examples,

the catalyst used was the catalyst in Example 7 of CN114433087A, and the catalyst was activated with hydrogen at 450°C for 3 hours before use.

The composition of the products was analyzed by chromatography.

The yield of hexamethylenediamine = the molar amount of hexamethylenediamine ÷ the molar amount of freshly fed hexanediol × 100%.

### Example 1

(1) Ammonia, hydrogen, 1,4-dioxane as a solvent and hexanediol were introduced into an ammoniation reactor filled with the catalyst to carry out an ammoniation reaction, wherein the molar ratio of ammonia to hexanediol was 28, the molar ratio of hydrogen to hexanediol was 3, the molar ratio of the solvent to hexanediol was 4, the temperature of the ammoniation reaction was 148°C, the reaction pressure was 12.5 MPaG, and the liquid phase volume space velocity of hexanediol was 0.45 h⁻¹.
(2) Ammonia and hydrogen in the reaction product were recovered by using a combination of flash evaporation and distillation: the ammoniation reaction product was sequentially subjected to three-stage vacuum flash evaporation, and the flash evaporation pressure was reduced gradually from 12 MPaG to 2 MPaG, wherein the pressures of the first stage of the flash evaporation to the third stage of the flash evaporation were set at 8 MPaG, 5 MPaG, and 2 MPaG, respectively. The gas phases at the top of three vacuum flash tanks were cooled to 40°C respectively, and then subjected to gas-liquid separation respectively, wherein the resultant liquid phase was returned to the vacuum flash tank at the respective stage respectively, and the resultant gas phase was pressurized and then returned to the ammoniation reactor. The liquid phase at the bottom of the third vacuum flash tank entered a deammoniation distillation column, which had a total of 11 theoretical plates and a top operating pressure of 1.1 MPaG. In order to save energy, the deammoniation distillation column was not equipped with a top condenser, and the liquid phase entered from the top of the deammoniation distillation column. The overhead stream (circulating ammonia) recovered from the deammoniation distillation column was sent back to the inlet of the ammoniation reactor, and the bottom stream was sent to a refining separation unit. Herein, the recovery rate of ammonia was 99.99%, and the recovery rate of hydrogen was 99.999%. The circulating hydrogen and ammonia contained 0.39wt% of hydrogen, 99.47wt% of ammonia, 0.12wt% of the solvent, 0.01wt% of water and 0.01wt% of hexamethyleneimine.

The liquid phase after distillation was sent to the refining separation unit, and was cut and separated with hexamethylenediamine as the key component according to different boiling points. The bottom stream of the deammoniation distillation column was subjected to a first separation to obtain a stream containing hexamethyleneimine, water and the solvent, followed by a second separation to obtain a hexamethylenediamine product (with a purity of 99.78wt%), a stream containing aminohexanol (withdrawn from the side line) and heavy components. Herein, the first separation was carried out in a first distillation column, and the conditions for operating the first distillation column included: a bottom temperature of 185°C, a top temperature of 50°C, a reflux ratio of 1.75, a top operating pressure of -0.08 MPaG, and a theoretical plate number of 19; the second separation was carried out in a second distillation column, and the conditions for operating the second distillation column included: a bottom temperature of 193°C, a top temperature of 132°C, a reflux ratio of 1.2, a top operating pressure of -0.09 MPaG, and a theoretical plate number of 68.

The stream containing hexamethyleneimine, water and the solvent was dehydrated by membrane separation method to obtain a dehydrated stream containing hexamethyleneimine and the solvent, wherein the conditions for operating the membrane separation device included: a membrane inlet pressure of 1.8 MPa, and a temperature of 38° C. The dehydrated stream containing hexamethyleneimine and the solvent had a water content of 0.19 wt %.

The stream containing aminohexanol was mixed with the dehydrated stream containing hexamethyleneimine and the solvent and then returned to the step (1) as a circulating stream, with a weight ratio of the circulating stream to fresh hexanediol being 4.3. Herein, the circulating stream comprised: 18.32 wt% of hexamethyleneimine, 16.38 wt% of aminohexanol, 3.87 wt% of hexanediol, 1.15 wt% of hexamethylenediamine and 56.75 wt% of the solvent.

The system was continuously operated to a stable state, and the molar yield of hexamethylenediamine was 88.4% without a solvent purification. The system had no blockage and could be operated continuously and smoothly for a long period of time.

### Example 2

(1) Ammonia, hydrogen, 1,4-dioxane as a solvent and hexanediol were introduced into an ammoniation reactor filled with the catalyst to carry out an ammoniation reaction, wherein the molar ratio of ammonia to hexanediol was 29, the molar ratio of hydrogen to hexanediol was 3.3, the molar ratio of the solvent to hexanediol was 3.5, the temperature of the ammoniation reaction was 158°C, the reaction pressure was 11.7 MPaG, and the liquid phase volume space velocity of hexanediol was 0.55 h⁻¹.
(2) Ammonia and hydrogen in the reaction product were recovered by using a combination of flash evaporation and distillation: the ammoniation reaction product was first depressurized to 2 MPaG, the gas phase at the top of the flash tank was first cooled to 40°C, and then subjected to gas-liquid separation, the resultant liquid phase was returned to the flash tank, and the resultant gas phase was pressurized and then returned to the ammoniation reactor. The liquid phase at the bottom of the flash tank entered a deammoniation distillation column, which had a total of 10 theoretical plates and a top operating pressure of 1 MPaG. In order to save energy, the deammoniation distillation column was not equipped with a top condenser, and the liquid phase entered from the top of the deammoniation distillation column. The overhead stream (circulating ammonia) of the deammoniation distillation column was sent back to the inlet of the ammoniation reactor, and the bottom stream was sent to a refining separation unit. Herein, the recovery rate of ammonia was 99.99%, and the recovery rate of hydrogen was 99.999%. The circulating hydrogen and ammonia contained 0.39wt% of hydrogen, 97.66wt% of ammonia, 1.55wt% of the solvent, 0.23wt% of water and 0.16wt% of hexamethyleneimine.

The bottom stream of the deammoniation distillation column was sent to the refining separation unit, and was cut and separated with hexamethylenediamine as the key component according to different boiling points. The bottom stream of the deammoniation distillation column was subjected to a first separation to obtain a stream containing hexamethyleneimine, water and the solvent, followed by a second separation to obtain a hexamethylenediamine product (with a purity of 99.7wt%), and followed by a third separation to obtain a stream containing aminohexanol and heavy components. Herein, the first separation was carried out in a first distillation column, and the conditions for operating the first distillation column included: a bottom temperature of 185°C, a top temperature of 51°C, a reflux ratio of 1.8, a top operating pressure of -0.08 MPaG, and a theoretical plate number of 22; the second separation was carried out in a second distillation column, and the conditions for operating the second distillation column included: a bottom temperature of 192°C, a top temperature of 135°C, a reflux ratio of 3.1, a top operating pressure of -0.09 MPaG, and a theoretical plate number of 25; and the third separation was carried out in a third distillation column, and the conditions for operating the third distillation column included: a bottom temperature of 215°C, a top temperature of 171°C, a reflux ratio of 2.8, a top operating pressure of -0.09 MPaG, and a theoretical plate number of 59.

The process of dehydrating the stream containing hexamethyleneimine, water and the solvent was: the stream containing hexamethyleneimine, water and the solvent was sent to a distillation column, a stream containing the solvent and water was obtained at the top of the column, and a stream containing hexamethyleneimine was obtained at the bottom of the column; the stream containing the solvent and water was then sent to a high-pressure distillation column, an azeotrope of the solvent and water was obtained at the top of the column, and a solvent stream was obtained at the bottom of the column; the azeotrope of the solvent and water was then sent to a low-pressure distillation column, the stream obtained at the top of the column was returned to the high-pressure distillation column, and the bottom stream (mainly composed of water) was discharged. After the dehydration treatment, the water removal rate for the stream containing hexamethyleneimine, water and the solvent was 99.2%, that is, the water content in the stream containing hexamethyleneimine and the solvent was 0.1wt%. Herein, the conditions for operating the distillation column included: a bottom temperature of 105°C, a top temperature of 55°C, a reflux ratio of 5, a top operating pressure of -0.075 MPaG, and a theoretical plate number of 22. The conditions for operating the high-pressure distillation column included: a bottom temperature of 229°C, a top temperature of 190°C, a reflux ratio of 0.85, a top operating pressure of 1.5 MPaG, and a theoretical plate number of 18. The conditions for operating the low-pressure distillation column included: a bottom temperature of 100°C, a top temperature of 79°C, a reflux ratio of 3, a top operating pressure of 0.05 MPaG, and a theoretical plate number of 16. In the example, the 1,4-dioxane solvent portion recovered from the bottom of the high-pressure distillation column was purified, and the purified solvent was also returned to the inlet of the reactor for recycling.

The stream containing aminohexanol was mixed with the dehydrated stream containing hexamethyleneimine and the solvent stream and then returned to the step (1) as a circulating stream, with a weight ratio of the circulating stream to fresh hexanediol being 5.1. Herein, the circulating stream comprised: 17.96 wt% of hexamethyleneimine, 15.97 wt% of aminohexanol, 3.78 wt% of hexanediol, 0.05 wt% of hexamethylenediamine and 61.75 wt% of the solvent.

The system was continuously operated to a stable state, and the molar yield of hexamethylenediamine was 88.6% with a solvent purification. The system had no blockage and could be operated continuously and smoothly for a long period of time.

### Example 3

(a) Ammonia, hydrogen and hexanediol were introduced into an ammoniation reactor filled with the catalyst to carry out an ammoniation reaction, wherein the molar ratio of ammonia to hexanediol was 29, the molar ratio of hydrogen to hexanediol was 1.4, the temperature of the ammoniation reaction was 140°C., the reaction pressure was 12.2 MPaG, and the liquid phase volume space velocity of hexanediol was 0.5 h⁻¹.
(b) Ammonia and hydrogen in the reaction product were recovered by using a combination of flash evaporation and distillation: the ammoniation reaction product was sequentially subjected to three-stage vacuum flash evaporation, and the flash evaporation pressure was reduced gradually from 12.2 MPaG to 2 MPaG, wherein the pressures of the first stage of the flash evaporation to the third stage of the flash evaporation were set at 8 MPaG, 5 MPaG, and 2 MPaG, respectively. The gas phases at the top of the three vacuum flash tanks were cooled to 40°C respectively, and then subjected to gas-liquid separation respectively, wherein the resultant liquid phase was returned to the vacuum flash tank at the respective stage respectively, and the resultant gas phase was pressurized and then returned to the ammoniation reactor. The liquid phase at the bottom of the third vacuum flash tank entered a deammoniation distillation column, which had a total of 10 theoretical plates and a top operating pressure of 1 MPaG. In order to save energy, the deammoniation distillation column was not equipped with a top condenser, and the liquid phase entered from the top of the deammoniation distillation column. The overhead stream (circulating ammonia) recovered from the deammoniation distillation column was sent back to the inlet of the ammoniation reactor, and the bottom stream was sent to a refining separation unit. Herein, the recovery rate of ammonia was 99.99%, and the recovery rate of hydrogen was 99.999%. The circulating hydrogen and ammonia contained 0.22 wt% of hydrogen, 99.73 wt% of ammonia, 0.02 wt% of water and 0.02 wt% of hexamethyleneimine.

The liquid phase after distillation was sent to the refining separation unit, and was cut and separated with hexamethylenediamine as the key component according to different boiling points. The bottom stream of the deammoniation distillation column was subjected to a first separation to obtain a stream containing hexamethyleneimine and water, followed by a second separation to obtain a hexamethylenediamine product (with a purity of 99.70 wt%), a stream containing aminohexanol (withdrawn from the side line) and heavy components. Herein, the first separation was carried out in a first distillation column, and the conditions for operating the first distillation column included: a bottom temperature of 182°C, a top temperature of 54°C, a reflux ratio of 1.0, a top operating pressure of 30 KPa, and a theoretical plate number of 25; the second separation was carried out in a second distillation column, and the conditions for operating the second distillation column included: a bottom temperature of 210°C, a top temperature of 132°C, a reflux ratio of 3.2, a top operating pressure of 20 KPa, and a theoretical plate number of 53.

The stream containing hexamethyleneimine and water was dehydrated by membrane separation method to obtain a dehydrated stream containing hexamethyleneimine, wherein the conditions for operating the membrane separation device included: a membrane inlet pressure of 2 MPa, and a temperature of 45°C. The dehydrated stream containing hexamethyleneimine had a water content of 0.48 wt %.

The stream containing aminohexanol was mixed with the dehydrated stream containing hexamethyleneimine and then returned to the step (1) as a circulating stream, with a weight ratio of the circulating stream to fresh hexanediol being 1.7. Herein, the circulating stream comprised: 47.07 wt% of hexamethyleneimine, 42.08 wt% of aminohexanol, 9.96 wt% of hexanediol, and 0.13 wt% of hexamethylenediamine.

The system was continuously operated to a stable state, and the molar yield of hexamethylenediamine was 86.1%. The devices and pipelines of the entire system needed to be insulated.

### Example 4

(a) Ammonia, hydrogen and hexanediol were introduced into an ammoniation reactor filled with the catalyst to carry out an ammoniation reaction, wherein the molar ratio of ammonia to hexanediol was 30, the molar ratio of hydrogen to hexanediol was 1.1, the temperature of the ammoniation reaction was 150°C, the reaction pressure was 12 MPaG, and the liquid phase volume space velocity of hexanediol was 0.45 h⁻¹.
(b) Ammonia and hydrogen in the reaction product were recovered by using a combination of flash evaporation and distillation: the ammoniation reaction product was sequentially subjected to three-stage vacuum flash evaporation, and the flash evaporation pressure was reduced gradually from 12 MPaG to 1 MPaG, wherein the pressures of the first stage of the flash evaporation to the third stage of the flash evaporation were set at 9 MPaG, 5 MPaG, and 1 MPaG, respectively. The gas phases at the top of the three vacuum flash tanks were cooled to 40°C respectively, and then subjected to gas-liquid separation respectively, wherein the resultant liquid phase was returned to the vacuum flash tank at the respective stage respectively, and the resultant gas phase was pressurized and then returned to the ammoniation reactor. The liquid phase at the bottom of the third vacuum flash tank entered a deammoniation distillation column, which had a total of 11 theoretical plates and a top operating pressure of 1.4 MPaG. In order to save energy, the deammoniation distillation column was not equipped with a top condenser, and the liquid phase entered from the top of the deammoniation distillation column. The overhead stream (circulating ammonia) recovered from the deammoniation distillation column was sent back to the inlet of the ammoniation reactor, and the bottom stream was sent to a refining separation unit. Herein, the recovery rate of ammonia was 99.99%, and the recovery rate of hydrogen was 99.999%. The circulating hydrogen and ammonia contained 0.40 wt% of hydrogen, 99.51 wt% of ammonia, 0.04 wt% of water and 0.04 wt% of hexamethyleneimine.

The liquid phase after distillation was sent to the refining separation unit, and was cut and separated with hexamethylenediamine as the key component according to different boiling points. The bottom stream of the deammoniation distillation column was subjected to a first separation to obtain a stream containing hexamethyleneimine and water, followed by a second separation to obtain a hexamethylenediamine product (with a purity of 99.70 wt%), a stream containing aminohexanol (withdrawn from the side line) and heavy components. Herein, the first separation was carried out in a first distillation column, and the conditions for operating the first distillation column included: a bottom temperature of 185°C, a top temperature of 56°C, a reflux ratio of 2.0, a top operating pressure of 28 Kpa (absolute pressure), and a theoretical plate number of 25; the second separation was carried out in a second distillation column, and the conditions for operating the second distillation column included: a bottom temperature of 208°C, a top temperature of 135°C, a reflux ratio of 3.5, a top operating pressure of 25 KPa (absolute pressure), and a theoretical plate number of 55.

The stream containing hexamethyleneimine and water was dehydrated by azeotropic distillation method to obtain a dehydrated stream containing hexamethyleneimine, wherein the conditions for operating the azeotropic distillation column included: a weight ratio of an azeotropic agent to the stream containing hexamethyleneimine and water of 51:1, a bottom temperature of 161°C, a top temperature of 90°C, a reflux ratio of 1, a top operating pressure of 0.1 MPaG, and a theoretical plate number of 22. The azeotropic agent was cyclohexane. The water content in the dehydrated stream containing hexamethyleneimine was less than 100 ppm.

The stream containing aminohexanol was mixed with the dehydrated stream containing hexamethyleneimine and then returned to the step (1) as a circulating stream, with a weight ratio of the circulating stream to fresh hexanediol being 2.55. Herein, the circulating stream comprised: 47.04 wt% of hexamethyleneimine, 41.86 wt% of aminohexanol, 9.90 wt% of hexanediol, and 0.12 wt% of hexamethylenediamine.

The system was continuously operated to a stable state, and the molar yield of hexamethylenediamine was 86.3%. The devices and pipelines of the entire system needed to be insulated.

### Example 5

(1) Ammonia, hydrogen, 1,4-dioxane as a solvent and hexanediol were introduced into an ammoniation reactor filled with the catalyst to carry out an ammoniation reaction, wherein the molar ratio of ammonia to hexanediol was 28, the molar ratio of hydrogen to hexanediol was 3.4, the molar ratio of the solvent to hexanediol was 3.2, the temperature of the ammoniation reaction was 159°C., the reaction pressure was 11.5 MPaG, and the liquid phase volume space velocity of hexanediol was 0.55 h⁻¹.
(2) Ammonia and hydrogen in the reaction product were recovered by using a combination of flash evaporation and distillation: the ammoniation reaction product was first depressurized to 2 MPaG, the gas phase at the top of the flash tank was first cooled to 40°C, and then subjected to gas-liquid separation, the resultant liquid phase was returned to the flash tank, and the resultant gas phase was pressurized and then returned to the ammoniation reactor. The liquid phase at the bottom of the flash tank entered a deammoniation distillation column, which had a total of 10 theoretical plates and a top operating pressure of 1 MPaG. In order to save energy, the deammoniation distillation column was not equipped with a top condenser, the liquid phase entered from the top of the deammoniation distillation column. The overhead stream (circulating ammonia) of the deammoniation distillation column was sent back to the inlet of the ammoniation reactor, and the bottom stream was sent to a refining separation unit. Herein, the recovery rate of ammonia was 99.99%, and the recovery rate of hydrogen was 99.999%. The circulating hydrogen and ammonia contained 0.64 wt% of hydrogen, 95.2 wt% of ammonia, 3.75 wt% of the solvent, 0.25 wt% of water and 0.16wt% of hexamethyleneimine.

The bottom stream of the deammoniation distillation column was sent to the refining separation unit, and was cut and separated with hexamethylenediamine as the key component according to different boiling points. The bottom stream of the deammoniation distillation column was subjected to a first separation to obtain a stream containing hexamethyleneimine, water and the solvent, followed by a second separation to obtain a hexamethylenediamine product (with a purity of 99.8wt%), and followed by a third separation to obtain a stream containing aminohexanol and heavy components. Herein, the first separation was carried out in a first distillation column, and the conditions for operating the first distillation column included: a bottom temperature of 182°C, a top temperature of 50°C, a reflux ratio of 1.8, a top operating pressure of -0.08 MPaG, and a theoretical plate number of 22; the second separation was carried out in a second distillation column, and the conditions for operating the second distillation column included: a bottom temperature of 192°C, a top temperature of 133°C, a reflux ratio of 3.0, a top operating pressure of -0.09 MPaG, and a theoretical plate number of 25; and the third separation was carried out in a third distillation column, and the conditions for operating the third distillation column included: a bottom temperature of 215°C, a top temperature of 175°C, a reflux ratio of 4.5, a top operating pressure of -0.09 MPaG, and a theoretical plate number of 59.

The stream containing hexamethyleneimine, water and the solvent was dehydrated by membrane separation method to obtain a dehydrated stream containing hexamethyleneimine and the solvent, wherein the conditions for operating the membrane separation device included: a membrane inlet pressure of 2.2 MPa, and a temperature of 48°C. The dehydrated stream containing hexamethyleneimine and the solvent had a water content of 0.39 wt %.

The stream containing aminohexanol was produced as a by-product and sent outside the battery limit. The dehydrated stream containing hexamethyleneimine and the solvent stream are returned to the step (1) as a circulating stream, with a weight ratio of the circulating stream to fresh hexanediol being 5.5. Herein, the circulating stream comprised: 23.04 wt% of hexamethyleneimine, 1.78 wt% of hexanediol, 0.08 wt% of hexamethylenediamine, and 75.1 wt% of the solvent.

The system was continuously operated to a stable state, and the molar yield of hexamethylenediamine was 84.3% with a solvent purification. The system had no blockage and could be operated continuously and smoothly for a long period of time.

The preferred embodiments of the invention are described in detail above. However, the invention is not limited thereto. Within the technical concept of the invention, a variety of simple modifications can be made to the technical solution of the invention, including a combination of various technical features in any other suitable manner. These simple modifications and combinations should also be regarded as the contents disclosed by the invention and are within the protection scope of the invention.

## Claims

1. A method for preparing hexamethylenediamine, which comprises the following steps:
(1) under ammoniation reaction conditions, optionally in the presence of a solvent, subjecting hexanediol and ammonia to an ammoniation reaction to obtain an ammoniation reaction product;
(2) separating and obtaining from the ammoniation reaction product a stream containing hexamethyleneimine and water, dehydrating the stream containing hexamethyleneimine and water to obtain a dehydrated stream containing hexamethyleneimine, and returning at least part of the dehydrated stream containing hexamethyleneimine as a circulating stream to the step (1), wherein a water content of the dehydrated stream containing hexamethyleneimine is less than 3 wt%, preferably less than 1.5 wt%.

2. The method according to claim 1, wherein the molar ratio of ammonia to hexanediol is 10-60:1, preferably 15-55:1; the ammoniation reaction is carried out in the presence of hydrogen, and the molar ratio of hydrogen to hexanediol is 0.05-8:1, preferably 0.4-5:1.

3. The method according to any one of claims 1-2, wherein the temperature of the ammoniation reaction is 100-220°C, preferably 110-210°C; the pressure of the ammoniation reaction is 5-20 MPaG, preferably 6-19 MPaG; the liquid volume space velocity of the fresh hexanediol is 0.05-10h⁻¹, preferably 0.07-5h⁻¹.

4. The method according to any one of claims 1-3, wherein the weight ratio of the circulating stream returned to the step (1) to the fresh hexanediol is 0.1-30:1, preferably 0.3-26:1.

5. The method according to any one of claims 1-4, wherein the dehydration includes at least one of membrane separation dehydration, azeotropic distillation dehydration, extractive distillation dehydration, adsorption dehydration, reverse osmosis dehydration, biological treatment dehydration and pressure swing distillation dehydration.

6. The method according to any one of claims 1-5, wherein the step (2) further comprises separating and obtaining a stream containing aminohexanol from the ammoniation reaction product, and returning the stream containing aminohexanol together with the dehydrated stream containing hexamethyleneimine as a circulating stream to the step (1);
preferably, the weight ratio of aminohexanol to hexamethyleneimine in the circulating stream is 0.1-8:1, for example 0.1-7:1.

7. The method according to any one of claims 1-6, wherein the approach for separating the ammoniation reaction product is: subjecting the ammoniation reaction product to a first separation to obtain the stream containing hexamethyleneimine and water, followed by a second separation to obtain a hexamethylenediamine product and a stream containing aminohexanol;
preferably, the first separation and the second separation each independently include at least one of distillation, membrane separation and pressure swing adsorption.

8. The method according to claim 7, wherein the step (2) further comprises pre-separating the ammoniation reaction product before separating the stream containing hexamethyleneimine and the hexamethylenediamine product in the ammoniation reaction product, to obtain circulating hydrogen and ammonia, wherein the circulating hydrogen and ammonia are returned to the ammoniation reaction; preferably, the pre-separation comprises at least one of flash evaporation, distillation and stripping.

9. The method according to any one of claims 1-8, wherein
the step (1) comprises: under the ammoniation reaction conditions, subjecting hexanediol and ammonia to the ammoniation reaction in the solvent to obtain the ammoniation reaction product;
the step (2) comprises: separating and obtaining from the ammoniation reaction product a stream containing hexamethyleneimine, water and the solvent, dehydrating the stream containing hexamethyleneimine, water and the solvent to obtain a dehydrated stream containing hexamethyleneimine and the solvent, and returning at least part of the dehydrated stream containing hexamethyleneimine and the solvent as the circulating stream to the step (1);
preferably, the solvent is at least one of tetrahydrofuran, 1,4-dioxane, n-hexane, cyclohexane, tert-butanol, tetramethylene sulfone and glycerol;
preferably, the weight ratio of the solvent to hexamethyleneimine in the circulating stream is 1-11:1, and/or a content of hexamethyleneimine in the circulating stream is 5-75wt%;
preferably, the weight ratio of the circulating stream to the fresh hexanediol is 0.1-24:1, preferably 0.4-21:1.

10. The method according to claim 9, wherein in the step (1), the molar ratio of ammonia to hexanediol is 18-55:1, preferably 22-43:1; the ammoniation reaction is carried out in the presence of hydrogen, and the molar ratio of hydrogen to hexanediol is 0.08-7:1, preferably 0.4-4:1.

11. The method according to any one of claims 9-10, wherein the temperature of the ammoniation reaction is 110-200°C, preferably 120-190°C; the pressure of the ammoniation reaction is 7-18 MPaG, preferably 8-14 MPaG, and the liquid volume space velocity of the fresh hexanediol is 0.05-8 h⁻¹, preferably 0.08-4 h⁻¹.

12. The method according to any one of claims 9-11, wherein the process of dehydrating the stream containing hexamethyleneimine, water and the solvent comprises: feeding the stream containing hexamethyleneimine, water and the solvent to a distillation column, obtaining a stream containing the solvent and water at the top of the column, and obtaining a stream containing hexamethyleneimine at the bottom of the column; feeding the stream containing the solvent and water to a high-pressure distillation column, obtaining an azeotrope of the solvent and water at the top of the column, and obtaining a solvent stream at the bottom of the column; and then feeding the azeotrope of the solvent and water to a low-pressure distillation column, and returning a stream obtained at the top of the column to the high-pressure distillation column; or
the process of dehydrating the stream containing hexamethyleneimine, water and the solvent comprises: subjecting the stream containing hexamethyleneimine, water and the solvent to a membrane separation dehydration at a membrane inlet pressure of 1-5 MPa and 15-200°C.

13. The method according to any one of claims 9-12, wherein the step (2) further comprises separating and obtaining the stream containing aminohexanol from the ammoniation reaction product, and returning the stream containing aminohexanol together with the dehydrated stream containing hexamethyleneimine and the solvent as the circulating stream to the step (1), wherein the weight ratio of aminohexanol to hexamethyleneimine in the circulating stream is 0.12-7:1.

14. The method according to any one of claims 1-8, wherein the step (1) comprises:
under the ammoniation reaction conditions, and in the absence of the solvent, subjecting hexanediol and ammonia to the ammoniation reaction to obtain the ammoniation reaction product;
preferably, the weight ratio of the circulating stream to the fresh hexanediol is 0.1-13:1,
preferably 0.3-10:1.

15. The method according to claim 14, wherein in the step (1), the molar ratio of ammonia to hexanediol is 18-50:1, preferably 22-38:1, and the molar ratio of hydrogen to hexanediol is 0.08-6:1, preferably 0.4-3:1.

16. The method according to claim 14 or 15, wherein the temperature of the ammoniation reaction is 120-210°C, preferably 130-200°C; the pressure of the ammoniation reaction is 7-17 MPaG, preferably 8-15 MPaG, and the liquid volume space velocity of the fresh hexanediol is 0.05-7h⁻¹, preferably 0.09-3.9h⁻¹.

17. The method according to any one of claims 14-16, wherein the step (2) further comprises separating and obtaining the stream containing aminohexanol from the ammoniation reaction product, and returning the stream containing aminohexanol together with the dehydrated stream containing hexamethyleneimine as the circulating stream to the step (1), wherein the weight ratio of aminohexanol to hexamethyleneimine in the circulating stream is 0.1-5:1.
